# EUROPEAN PATENT SPECIFICATION

(11) **EP 2 514 319 B1**
(45) Date of publication and mention of the grant of the patent: **23.09.2015**
(21) Application number: 12172524.6
(22) Date of filing: 02.12.2010
(51) Int. Cl.: A23K 1/16, A23K 1/18

(54) **Methods and feed compositions for masking of fish semiochemicals**
Verfahren und Futterzusammensetzungen zur Maskierung von Fischbotenstoffen
Procédés et compositions d'alimentation pour masquer des produits sémiochimiques de poisson

(30) Priority: 02.12.2009 NO 20093460
(43) Date of publication of application: 24.10.2012
(62) Divisional of application: 10803171.7
(73) Proprietor: Ewos Innovation AS, 4335 Dirdal (NO)
(72) Inventor: Wadsworth, Simon, 9405 Harstad (NO); Vecino, José Luis González, 4326 Sandnes (NO); Pino, Jorge, Puerto Varas (CL); Mordue, Jenny, AB15 9AQ Aberdeen (GB)
(74) Representative: Acapo AS

(56) References cited:
- EP-A1- 1 208 751
- WO-A2-2004/091307
- US-A- 5 139 791
- US-A- 5 504 081
- US-A1- 2005 266 052
- ROTH M ET AL: "Current practices in the chemotherapeutic control of sea lice infestations in aquaculture: A review", JOURNAL OF FISH DISEASES, OXFORD, GB, vol. 16, no. 1, 1 January 1993 (1993-01-01), pages 1-26, XP009146063, ISSN: 0140-7775

## Description

### FIELD OF THE INVENTION

The present invention relates to an extract of lavender for masking the odor of fish semiochemicals in water, wherein the attraction between a parasite and a fish in said water is reduced. The invention also relates to a lavender extract for reducing the attraction between a parasite and a fish. The invention also relates to the use of an extract for the prevention and/or treatment of a parasite infection in fish.

### BACKGROUND TO THE INVENTION

Sea lice (*Lepeophtheirus salmonis*, *Caligus* sp.) are the major pathogen currently affecting the global salmon farming industry and have a significant impact on many areas of production. Economic impact on the aquaculture industry is high due to high annual losses. There is also continued concern over the impact of salmon farming on wild salmon populations with increased density of sea lice adjacent to these production sites. Control measures have been reliant upon the use of a number of chemotherapeutants since the 1970's. Reduced efficacy has now been reported for all compounds, with the exception of the insect growth regulators (IGR) diflubenzuron and teflubenzuron. Further methods are therefore required to effectively control sea lice, in conjunction with sea lice medicines.

### Host-specific parasites

The *Lepeophtheirus* genus of sea lice is a host-specific parasite. *L. salmonis* will only complete its life cycle on salmonid species, although mobile stages may occasionally be observed as opportunists on additional fish types. Other *Lepeophtheirus* sp. will target a narrow range of other fish species.

### Immune suppression of the host

The *Lepeophtheirus* genus of sea lice has evolved a range of mechanisms to suppress the immune response of their particular hosts. To overcome a potentially fatal inflammatory reaction the sea lice release a series of secretary / excretory products (SEP) into the host tissue, via salivary glands. Prostaglandins (PGE₂), alkaline phosphatase and a range of trypsin-like proteases have been identified as sea lice SEPs. It is thought that several additional unidentified factors such as phosphatase, apyrase and macrophage inhibition factor are also present.

### Effect of immune suppressants

*L. salmonis* has a significant immunosuppressive effect on a range of responses in Atlantic salmon including reduced respiratory burst, lower macrophage activity, increased apoptosis, necrosis, decreased numbers of mucosal cells and down-regulation of immune genes such as interleukin IL-1ß and MHC-1. Suppression occurs at localised attachment sites, although a more generalised effect may occur with higher levels of sea lice infection. Once they have suppressed the immune system of the host, the lice are able to extend a frontal filament for a secure attachment. This is intimately associated with the host tissues and able to survive any subsequent immune response from that species.

### A fatal risk of attaching to the wrong host

*Lepeophtheirus* sp. are not able to suppress the immune system of non-host species. If lice try and settle on to a resistance fish species the immune response will kill it. Thus correct identification of the host is essential for attachment and survival of *Lepeophtheirus* sp.

### Correct host identification

Sea lice have advanced olfactory and contact chemoreceptors that are capable of accurate identification of specific host molecules. Semiochemicals (behaviour-modifying chemicals) are used by a range of arthropods in chemical communication systems to locate a host, mate or oviposition site. Similarly, many copepods use chemical cues to identify and seek out mates.

### Caligus species

Lice within the *Caligus* genus have an extensive range of potential hosts; C *elongatus* is known to infect over 80 host species world vide. *Caligus* have been found to posses a greater range and quantity of serine and non-serine proteases than *L. salmonis* and this may assist in defeating a greater range of immune responses from many different species. In addition *Caligus* deploy a different attachment mechanism that is not as intimately associated with host tissue. *Caligus* remove the epidermal tissue from the scales and then the frontal filament attaches directly to the cleared scales via a basal plate. The frontal filament is much longer than that deployed by *L. salmonis* and this allows the louse to remain at some distance from the host immune system. Despite these generalists adaption's some *Caligus* species still demonstrate a high degree of host specificity. This may develop in populations in areas where a particular host population is abundant such as *Caligus rogercresseyi* which are now the dominant sea lice species on salmon farms in Chile.

Through behavioral trials, tested the hypothesis that the inter-and intraspecific relationships of salmon louse, *C*. *rogercresseyi* are mediated by semiochemical compounds has been tested. It has been shown that the host species studied, Coho salmon, Atlantic salmon, and Rainbow trout, emit chemical signals that attract sea lice.

The object of the present invention is to provide an extract for prevention and control of sea lice attraction to, and infections in fish, preferable Salmonidae that is easily applicable, effective in long-term use and are considered as environmentally friendly and less toxic than many known chemotherapeutants. In particular, an object of the present invention is to provide a feed composition and an extract for masking the semiochemical compounds in order to reduce the attraction of a sea lice for Salmonidae.

EP1208751 discloses natural active substances such as lavender oil against fish parasites.

WO2004/091307 discloses lavender oil in feed of aquatic animals for the control of parasites.

### SUMMARY OF THE INVENTION

A first aspect of the present invention relates to an extract of lavender for use in masking the odor of a fish semiochemical in water, wherein the attraction between a parasite and a fish in said water is reduced, characterized in that an extract of lavender is added to said water or is administered to a fish in said water.

Preferably, said fish semiochemical is isophorone, preferable 1-Octen-3-ol or 6-methyl-5-hepten-2-one.

Preferably, said fish is a Salmonidae, preferable selected from the group consisting of Atlantic salmon, Coho salmon, Chinook, rainbow trout and Arctic charr.

Preferably, said water is Salmonidae conditioned sea water or said fish in the water is a Salmonidae.

Preferably, the extract reduces the attraction between a parasite and said fish.

Preferably, said Salmonidae is Atlantic salmon.

Preferably, said Salmonidae is rainbow trout.

Preferably, said parasite is an ectoparasite, preferable sea lice (*Lepeophtheirus salmonis*, *Caligus* sp.).

Preferably, the compound or extract is used for the manufacturing of a pharmaceutical or nutraceutical composition, or functional food.

### DESCRIPTION OF THE INVENTION

Embodiments of the invention will now be described, by the way of examples with reference to the following figures:

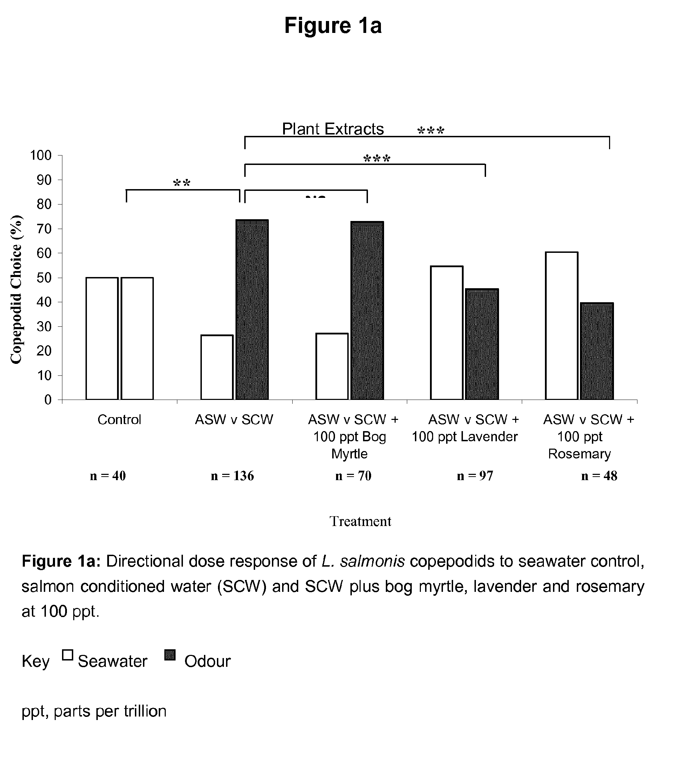

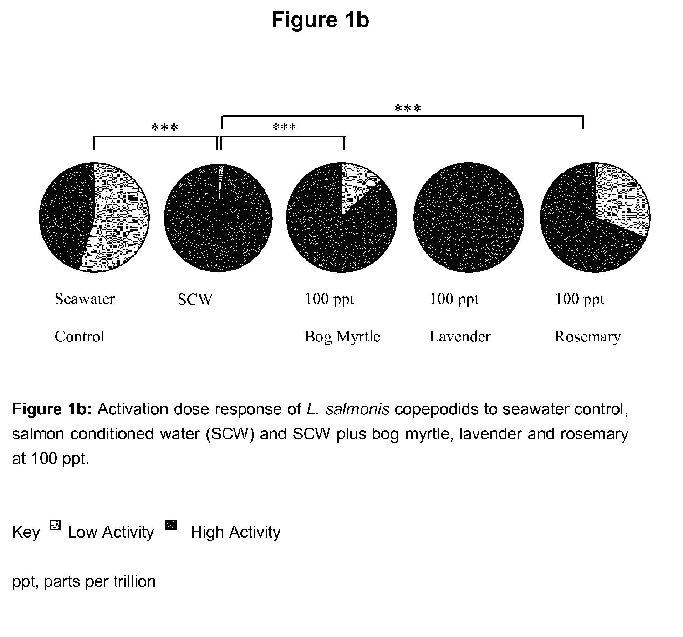

Figure 1 a shows the directional dose response of *L. salmonis* copepodids to seawater control, salmon conditioned water (SCW) and SCW plus bog myrtle, lavender and rosemary at 100 ppt.
Figure 1 b shows the activation dose response of *L. salmonis* copepodids to seawater control, salmon conditioned water (SCW) and SCW plus bog myrtle, lavender and rosemary at 100 ppt.
Figure 2a shows the directional dose response of *L. salmonis* copepodids to seawater control, salmon conditioned water (SCW) and SCW plus 100 and 1000 ppt lavender.
Figure 2b shows the activation dose response of *L. salmonis* copepodids to seawater control, salmon conditioned water (SCW) and SCW plus 100 and 1000 ppt lavender.

### EXPERIMENTAL SECTION

### Example 1: In vitro assessment of the effect of different compounds on Lepeophtheirus salmonis

A number of plant products were tested for their ability to mask salmon odour in order to inhibit the attractant of lice to salmon and to prevent *L. salmonis* settlement on salmon. A Y-tube behavioural arena was developed and used to test the ability of plant extracts/compounds to inhibit copepodid attraction to salmon conditioned water.

Products tested were:
- plant extracts; bog myrtle, lavender, rosemary

### Material and methods:

### Lice collection

Ovigerous female *Lepeophtheirus salmonis* were collected from Atlantic salmon. Material was transported on ice to the laboratory with clean seawater for sorting. Water from the source site was collected and used for subsequent rearing of egg strings. Strings were removed gently from their point of attachment to adult females using ultra-fine forceps and placed in 2 L glass conical flasks. All flasks were aerated to keep the strings in suspension and promote hatching. Egg strings were reared under a 16 h light - 8 h dark regime and at 12°C ambient temperature in water from the source site.

Development of the egg to the copepodid was determined as a function of the mean temperature following Johnson and Albright (1991). Strings were monitored twice daily for hatching of nauplii and subsequent development to the copepodid stage, at which point they were removed for use in behavioural bioassays.

### Fish Conditioned Water

Fish conditioned water was collected as described by Devine *et al.* (2000) and Ingvarsdottir *et al.* (2002b). Atlantic salmon, S. *salar* were maintained in aquaria containing artificial seawater (32 ‰). Fish conditioned water was obtained by placing the fish for 24 h into a circulating flume (20 cm x 25 cm x 420 cm) filled with artificial seawater (100 L) circulated at a rate of 30 cm s⁻¹. Aeration was provided by bubbling compressed air into the raceway. Standardisation of fish odour in the water was achieved by using the water at a concentration of 8-10 g live fish L⁻¹ 24 h⁻¹. Conditioned water was either used immediately or frozen for later use.

### Lice Behaviour L. salmonis

A vertical Y-tube bioassay modified by Bailey *et al.* (2006) from that previously described by Ingvarsdóttir *et al.* (2002a) was used to study *L. salmonis* copepodid activation and directional (taxis) responses to host semiochemical components and potential host-masking compounds. The Y-tube was constructed from glass (1 cm diameter bore) moulded into a 'Y' design between two glass sheets of glass (2 mm thick). The arms were 6.5 cm in length and the main leg was 8 cm long. The main leg of the Y-tube was fitted with a glass stopper and filter to prevent copepodids from entering the outflow tubing running to waste. A syringe pump (SP 200 iz, World Precision Instruments, Florida, USA) held two plastic 60 mL syringes (Terumo Monoject, New Jersey, USA), which were loaded with test odours prior to use. The syringe pump was programmed to deliver a consistent flow rate of 2 mL min⁻¹. Chemical dyes demonstrated a clear demarcation of the flow down each arm and no mixing of water in the main leg of the T-tube.

When single chemical stimuli were tested e.g. salmon conditioned water (SCW), the test water was introduced to one arm whilst artificial seawater (ASW) at 32 ‰ was introduced into the other. When one of the isothiocyanates for example was tested, seawater was introduced into one arm whilst SCW plus the isothiocyanate at the desired concentration were introduced to the other. The introduction of stimuli was alternated between left and right inflow arms during each experiment, with washing in between, to eliminate positional bias. At the beginning of each experiment, the Y-tube was hallowed to fill and run with seawater or seawater plus a cue/masking chemical, and a single copepodid was introduced using PTFE tubing and syringe into the tube at a point 1.5 cm above the base of the main leg. The copepodid was allowed a maximum of 3 min to respond. Each trial consisted of 1 copepodid.

Replicate tests were carried out over a period of four days to monitor for age effects of the lice on results.

Behaviour was defined by the degree of movement within the Y-Tube, as described by Ingvarsdóttir *et al.* (2002b). Behaviour was divided into two categories, low and high. Low activity was defined as the movement of the copepodid less than the length of the main leg. High activity was defined as movement of the copepodid more than the length of the main leg. Movement into either arm was also regarded as high activity. Both activation and directional responses of copepodids were measured. For directional responses, the number of copepodids choosing the stimulus arm rather than the control arm within the allocated 3 min period were compared to the control in which seawater was presented in both arms.

### Chemicals

Chemicals used in behavioural bioassays were supplied by the Chemical Ecology Group at Rothamsted Research, Harpenden, Hertfordshire, UK. Solutions of individual chemicals in ethanol (0.001, 0.01, 0.1 and 1 mg/mL) were prepared and diluted to 1 µL/L in artificial seawater (Ingvarsdóttir *et al*., 2002b) to give a final concentration of 0.1, 1, 10, 100 and 1,000 parts per trillion (ppt) respectively.

### Data Analysis

Copepodid responses to ASW (Artificial sea water) and SCW (Salmon conditioned sea water) across all experiment days were compared in the first instance using a chi-square test to determine if there was a day effect on louse behaviour. If this proved to be non-significant, it implies that the data are consistent across days and therefore can be pooled.

For directional responses and experiments on activity, the null hypothesis that all lice in all treatments behaved the same was tested using a 'global' χ² contingency table (Zar, 1999). Upon rejection of that hypothesis, data were analysed by *post hoc* targeted pairwise comparisons using a 2 x 2 χ² contingency table (Zar, 1999) to identify whether pairs of treatments of interest were significantly different.

### Results in vitro assessment Lepeophtheirus salmonis

### Plant Extracts

The global χ² showed that lice did not behave the same in all treatments (χ² = 33.38, df = 4, P < 0.001) in directional response assays. When compared with the seawater control, significantly more copepodids chose the arm containing the salmon conditioned water, SCW (χ² = 7.89, df = 1, P < 0.01). A significant decrease in copepodid responses was detected with SCW plus 100 ppt lavender (χ² = 19.03, df = 1, P < 0.001) and 100 ppt rosemary (χ² = 17.89, df = 1, P < 0.001) when compared against SCW responses. However, no difference in directional responses was detected between SCW plus 100 ppt bog myrtle and SCW responses (χ² = 0.01, df = 1, NS; Figure 1 a). The number of *L. salmonis* copepodids making directional responses, not choosing and the total number of replicates for each treatment are presented in Table 1 a.

**Table 1a**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 40 | 113 | 153 |
| ASW v SCW | 136 | 15 | 151 |
| ASW v SCW + 100 ppt Bog Myrtle | 70 | 30 | 100 |
| ASW v SCW + 100 ppt Lavender | 97 | 4 | 101 |
| ASW v SCW + 100 ppt Rosemary | 48 | 52 | 100 |

The global χ² showed that lice did not behave the same in all activity treatments (χ² = 144.34, df = 4, P < 0.001). When compared with the seawater control, a significant increase in high activity was detected with SCW (χ² = 91.70, df = 1, P < 0.001). Significantly more copepodids showed low activity with SCW plus 100 ppt bog myrtle (χ² = 12.23, df = 1, P < 0.001) and SCW plus 100 ppt rosemary (χ² = 43.24, df = 1, P < 0.001) when compared against SCW responses. No difference in activity was detected between SCW plus 100 ppt lavender however (χ² = 2.03, df = 1, NS; Figure 1 b).

### Lavender Dose Response

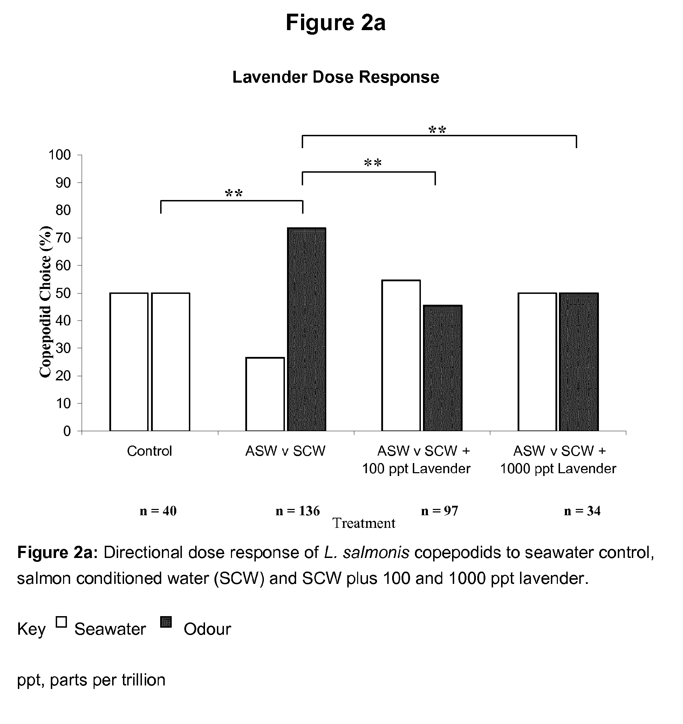

The global χ² showed that lice did not behave the same in all treatments (χ² = 19.46, df = 3, P < 0.001) in directional response assays. When compared with the seawater control, significantly more copepodids chose the arm containing the salmon conditioned water, SCW (χ² = 7.89, df = 1, P < 0.01). A significant decrease in copepodid responses was detected when SCW plus 100 ppt (χ² = 19.03, df = 1, P < 0.001) and 1,000 ppt lavender (χ² = 7.02, df = 1, P < 0.01) were compared against SCW responses (Figure 2a). The number of *L. salmonis* copepodids making directional responses, not choosing and the total number of replicates for each treatment are presented in Table 1 b.

**Table 1b**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 40 | 113 | 153 |
| ASW v SCW | 136 | 15 | 151 |
| ASW v SCW + 100 ppt Lavender | 97 | 4 | 101 |
| ASW v SCW+ 1,000 ppt Lavender | 34 | 2 | 36 |

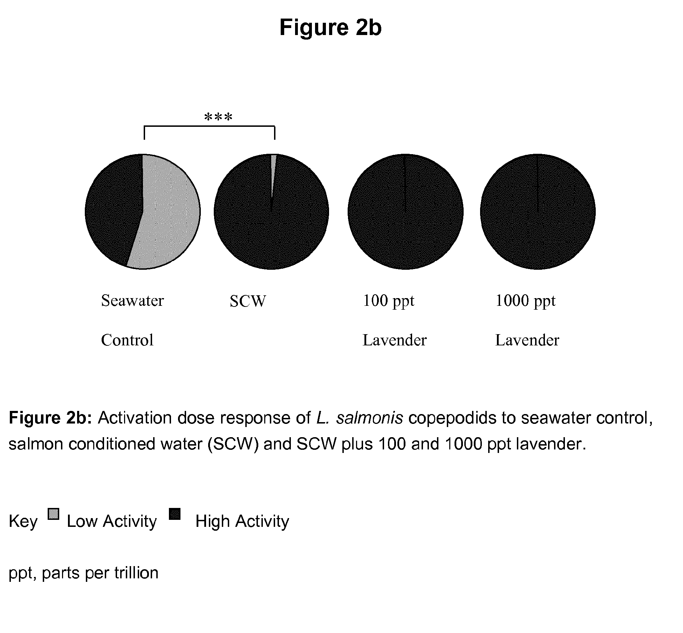

The global χ² showed that lice did not behave the same in all activity treatments (χ² = 160.36, df = 3, P < 0.001). When compared with the seawater control, a significant increase in high activity was detected with SCW (χ² = 91.70, df = 1, P < 0.001). No difference in activity was detected between SCW plus 100 ppt (χ² = 2.03, df = 1, NS) and 1,000 ppt lavender (χ² = 0.81, df = 1, NS; Figure 2b) however.

### Discussion:

In this study, it has been shown that copepodid larvae of the salmon louse, *L*. *salmonis*, show significant directional responses to isophorone, a component of salmon conditioned water. Isophorone has been identified as a behaviourally active component of salmon-conditioned water (Bailey *et al*., 2006) and was therefore used as a host cue to elicit a response in preliminary experiments.

For the plant extracts, both rosemary and lavender at 100 parts per trillion were effective at masking the salmon conditioned water. Bog myrtle dose response assays showed significant masking to occur at the 1,000 ppt concentration however.

A high number of non-choosers were seen in all seawater controls and is due to a lack of cues to stimulate a behavioural response from the lice.

In general, the seawater controls showed predominantly low activity behaviour in copepodids. This switched to high activity in the presence of a positive cue i.e. either isophorone or salmon conditioned water. Low activity re-appeared in the profile when test compounds were introduced, suggesting that the chemicals masked the effect of the isophorone or salmon conditioned water in copepodids. The extent of masking was variable between compounds and is thought to be related to the original field source of *L. salmonis.*

### Conclusions from example 1:

The use of plant derived masking compounds has been shown to significantly disrupt *L. salmonis* copepodid attraction to host (salmon) conditioned water *in vitro.* By masking the profile of the key host recognition molecules it was surprisingly possible to significantly reduce the host response of both *L. salmonis* and *C*. *rogercresseyi.* In the shown series of Y-tube assessments, sea lice showed a significant activity towards host odours from control Atlantic salmon. Inclusion of a series of masking compounds of vegetable origin effectively reduced this response in both species. Diallyl sulphide, diallyl disulphide, butyl isothiocyanate, allyl isothiocyanate, propyl Isothiocyanate, rosemary oil, lavender oil and bog myrtle were identified as candidate compounds for masking salmon host compounds.

The following compounds and concentrations were especially promising: Diallyl sulphide (10parts per trillion), diallyl disulphide (1 00parts per trillion), butyl isothiocyanate (100 parts per trillion), propyl isothiocyanate (100 parts per trillion) rosemary oil (100 parts per trillion), lavender oil (100 parts per trillion) and bog myrtle (1,000 parts per trillion).

### Definitions of terms:

The term "semiochemical" (semeon means a signal in Greek) is a generic term used for a chemical substance or mixture that carries a message. These chemicals acts as messengers for members of the same species or in some cases other species. It is usually used in the field of chemical ecology to encompass pheromones, allomones, kairomones, attractants and repellents. Please note especially that the term in respect of this application is not restricted to messengers between the same species, and that the term specifically is used to denote messengers between different species, such as between a Salmonidae and a parasite. The term is intended to include the chemical compounds which are specific for the attraction of parasites to Salmonidae, and especially to the attraction of sea lice to Salmonidae.

## Claims

1. An extract of lavender for use in masking the odor of a fish semiochemical in water, wherein the attraction between a parasite and a fish in said water is reduced, **characterized in that** said extract of lavender is added to said water or is administered to a fish in said water.

2. Extract according to claim 1, wherein said fish semiochemical is isophorone, preferably 1-Octen-3-ol or 6-methyl-5-hepten-2-one.

3. Extract according to claim 1, wherein said fish is a Salmonidae, preferable selected from the group consisting of Atlantic salmon, coho salmon, Chinook, rainbow trout and Arctic charr.

4. Extract according to claim1, wherein said water is Salmonidae conditioned sea water or said fish in the water is a Salmonidae.

5. Extract according to claim 4, wherein said Salmonidae is Atlantic salmon.

6. Extract according to claim 4, wherein said Salmonidae is rainbow trout.

7. Extract according to claim 5, wherein said parasite is an ectoparasite.

8. Extract according to claim 7, wherein said ectoparasite is sea lice (*Lepeophtheirus salmonis, Caligus* sp.).

## Patentansprüche

1. Extrakt aus Lavendel zur Verwendung in der Maskierung des Geruchs einer Fisch-Semiochemikalie in Wasser, wobei die Anziehung zwischen einem Parasit und einem Fisch in dem Wasser verringert wird, **dadurch gekennzeichnet, dass** der Extrakt aus Lavendel dem Wasser zugesetzt wird oder an einen Fisch in dem Wasser verabreicht wird.

2. Extrakt nach Anspruch 1, wobei die Fisch-Semiochemikalie Isophoron, vorzugsweise 1-Octen-3-ol oder 6-Methyl-5-hepten-2-on ist.

3. Extrakt nach Anspruch 1, wobei der Fisch ein Salmonide ist, vorzugsweise ausgewählt aus der Gruppe, bestehend aus Atlantischer Lachs, Silberlachs, Königslachs, Regenbogenforelle und Seesaibling.

4. Extrakt nach Anspruch 1, wobei das Wasser für Salmoniden konditioniertes Seewasser oder der Fisch in dem Wasser ein Salmonide ist.

5. Extrakt nach Anspruch 4, wobei der Salmonide ein Atlantischer Lachs ist.

6. Extrakt nach Anspruch 4, wobei der Salmonide eine Regenbogenforelle ist.

7. Extrakt nach Anspruch 5, wobei der Parasit ein Ektoparasit ist.

8. Extrakt nach Anspruch 7, wobei der Ektoparasit die Fischlaus (Lepeophtheirus salmonis, Caligus sp.) ist.

## Revendications

1. Extrait de lavande destiné à être utilisé dans le masquage d'un sémiochimique de poisson dans l'eau, où l'attraction entre un parasite et un poisson dans la dite eau est réduite, **caractérisé en ce que** le dit extrait de lavande est ajouté à la dite eau ou est administré à un poisson dans la dite eau.

2. Extrait selon la revendication 1, **caractérisé en ce que** le dit sémiochimique de poisson est de l'isophorone, de préférence le 1-Octen-3-ol ou le 6-Méthyl-5-heptène-2-one.

3. Extrait selon la revendication 1, **caractérisé en ce que** le dit poisson est un salmonidé, de préférence choisi dans le groupe composé du saumon de l'Atlantique, du saumon coho, du saumon royal, de la truite arc-en-ciel et de l'omble de l'Arctique.

4. Extrait selon la revendication 1, **caractérisé en ce que** la dite eau est de l'eau de mer conditionnée pour les salmonidés ou que le dit poisson dans l'eau est un salmonidé.

5. Extrait selon la revendication 4, **caractérisé en ce que** le dit salmonidé est du saumon de l'Atlantique.

6. Extrait selon la revendication 4, **caractérisé en ce que** le dit salmonidé est de la truite arc-en-ciel.

7. Extrait selon la revendication 5, **caractérisé en ce que** le dit parasite est un ectoparasite.

8. Extrait selon la revendication 7, **caractérisé en ce que** le dit ectoparasite est un pou de mer (*Lepeophthéirus salmonis, Caligus* sp.).
